# EUROPEAN PATENT SPECIFICATION

(11) **EP 4 454 572 B1**
(45) Date of publication and mention of the grant of the patent: **22.04.2026**
(21) Application number: 22910260.3
(22) Date of filing: 10.11.2022
(51) Int. Cl.: A61B 17/115, A61B 17/068, A61B 17/11, A61B 17/064, A61B 17/00, A61B 90/00

(54) **INSTRUMENT FOR SURGICAL STAPLING BETWEEN A VASCULAR PROSTHESIS AND THE AORTA**
CHIRURGISCHES KLAMMERINSTRUMENT ZWISCHEN EINER GEFÄSSPROTHESE UND DER AORTA
INSTRUMENT D'AGRAFAGE CHIRURGICAL ENTRE UNE PROTHÈSE VASCULAIRE ET L'AORTE

(30) Priority: 20.12.2021 ES 202132499 U
(43) Date of publication of application: 30.10.2024
(73) Proprietor: Fundación Para la Investigación del Hospital Universitario y Politécnico La Fe de la Comunidad Valenciana, 46025 Valencia (ES)
(72) Inventor: MIRALLES HERNÁNDEZ, Manuel, 46026 Valencia (ES)
(74) Representative: Clarke, Modet y Cía., S.L.
(86) International application number: PCT/ES2022/070725
(87) International publication number: WO 2023/118626

(56) References cited:
- WO-A1-2010/054404
- WO-A2-2005/089059
- WO-A2-2005/115254
- ES-U- 1 075 401
- US-A- 4 310 115
- US-A- 5 188 638
- US-A- 5 346 115
- US-A- 5 720 755
- US-A1- 2013 264 371

## Description

### TECHNICAL FIELD

The present invention relates to an instrument for surgical stapling between a vascular prosthesis and the aorta by means of an eversion technique and internal overlapping between the two. This device allows to perform the anastomosis or aortic attachment, in an open or laparoscopic way, with greater ease and safety than the devices known in the prior art by means of a combination of stapling elements. The instrument for vascular surgical stapling for aortic anastomosis, object of the invention, is applicable in the healthcare industry.

### BACKGROUND OF THE INVENTION

The development of terminally-terminal and terminally-lateral automatic or semi-automatic suturing systems of tubular structures (intestine, bile duct,...) has numerous antecedents and designs in surgery in recent decades. However, its introduction in the field of vascular sutures has been limited by various factors, such as being subjected to the pulsatile flow of blood at high pressure or the presence of wall irregularities (atheroma plaques) of different consistency (fibrosis, lipids, calcium). That is why, at least in part, drum-based designs with circular suture lines (common in digestive surgery) have had limited application and scarce diffusion.

The method of manual suturing (eversion with internal aortic wall overlap) has been developed in recent years, alone or associated with aortic balloon occlusion. It is especially useful in the preparation of the anastomosis or proximal union between the prosthesis and the aorta, during the aortic surgery of the juxta-renal aneurysm, although it does not exclude other locations. The development of a prototype aortic suture as the object of the invention allows its adaptation to laparoscopic surgery of the aorta (abdominal or thoracic).

US 5 188 638 A relates to surgical suturing devices and methods for applying an anastomosis onto hollow organs, e.g. the organs of the digestive tract or blood vessels, for instance, for joining them together in end-to-end or end-to-side utilizing fastener securing techniques.

WO 2010/054404 A1 relates to a device for acquiring and stapling multiple tissue regions within a subject's stomach using a multi-fire staple head with a rotary cartridge.

### SUMMARY OF THE INVENTION

The present invention is defined by the features of the independent claim. Embodiments of the invention are defined in the dependent claims.

An object of the invention is an instrument for surgical stapling between a vascular prosthesis and the aorta, comprising a handle, a stem associated with the handle, a head associated with the stem, a ring associated with the stem, anterolateral staples and posterior staples.

The handle comprises an ergonomic shell with a trigger configured to give two instructions, a first one of compression of the tissues, with a first travel of the trigger, and a second one of ejection of the anterolateral staples two by two, with a second travel of the trigger; a head position lock pushbutton to lock the head in a position with respect to the ring; an anchoring/release selector of the head with respect to the ring to release the fixation between the head and the ring; and an emergency release button to activate a decompression system.

The stem comprises a shaft and internal transmission means for translating the instructions given by the handle to the head.

The head comprises a body where a rotary magazine is housed which, in turn, has a plurality of perimeter housings, configured to house the anterolateral staples before being placed in their working position for stapling and through which said anterolateral staples come out when they are placed in their stapling position; an anchoring mechanism to the ring to fix the head to the ring; a compression-decompression mechanism to displace the body by performing a homogeneous compression of the tissues to be stapled, prior to the completion of a firing of anterolateral staples; and a firing mechanism of the anterolateral staples to actuate the rotary magazine to perform the firing of two anterolateral staples; means to provide a rotating movement to the rotary magazine; a rotating joint to provide a rotating movable joint between the stem and the head itself.

The ring comprises a cylindrical body with an inner conduit for housing the vascular prosthesis therein, facilitating the eversion of its proximal end and its overlapping with the internal surface of the aorta. A plurality of perimeter holes configured to receive the legs of the anterolateral staples and provide them with the shape of a B; an anchoring port for fixing them to the head; a stapling mechanism for the posterior aortic sector, which comprises a stapling sheet with a plurality of through holes, the posterior staples and a means for expelling them that act to push them out of the ring until they contact the stapling sheet through the central zone; this causes them to deform by acquiring the closed shape that compresses the vascular prosthesis and the wall of the aorta located on the stapling sheet between its limits, thus fixing the vascular prosthesis to the aorta through the stapling sheet and the posterior staples.

In the instrument, object of the invention, the means for ejecting the posterior staples, incorporated in the ring, comprise an actuating stem for displacing a series of sliding wedges to drive a series of plates that eject the posterior staples, where the sliding wedges are associated with retaining flaps, which describe a backward movement to release the stapling sheet from its fixation with respect to the stapling mechanism for the posterior aortic sector.

The instrument, object of the invention, includes an auxiliary gripper comprising a pull for actuating the stapling mechanism for the posterior aortic sector incorporated in the ring.

In the instrument, object of the invention, the auxiliary gripper comprises an adapter at its distal end that surrounds a fastening cannula located in the ring itself, such that the fastening cannula contains a driving cylindrical element that activates the stapling mechanism for the posterior aortic sector incorporated in the ring.

In the instrument, object of the invention, the stapling sheet, incorporated in the ring, comprises two through holes configured to incorporate additional sutures if necessary.

In the instrument, object of the invention, the firing mechanism comprises a driven gear mounted on a shaft, which displaces a runner, provided with a telescopic cam shaft and a limiting cam shaft, such that the runner, through a coupling shaft, allows the rotation of a fork that is associated with a drive pawl, mounted on a shaft and provided with a return spring, which promotes the rotation of the rotary magazine, such that the runner has a last section where, once the rotation is completed and the correct positioning of the rotary magazine is ensured by means of a fixer, the telescopic cam shaft abuts the runner itself, and is dragged along with it until said telescopic cam shaft exerts the necessary pressure, ultimately promoting the activation of firing levers that displace rack skids, such that these rack skids displace geared levers that displace sliding plates that eject the anterolateral staples for their B-shaped deformation.

In the instrument, object of the invention, the head comprises a complementary mechanism, associated with the firing mechanism, which comprises a spring configured to displace a friction wedge, associated by means of a shaft to a dynamic stop, which is attached by means of an oscillating pivot to the fixer that determines the position of the firing mechanism by arranging it for firing and allowing unlocking, dynamic travel stop and locking movements.

In the instrument, object of the invention, the compression-decompression mechanism comprises driven racks joined together by joining columns configured to rotate traction eccentrics, providing an advance movement of the head to exert the compression of the tissues to be stapled; it also comprises a stop cam to limit the decompression movement.

In the instrument, object of the invention, the anchoring mechanism between the head and the ring comprises driven gears, joined together by an integral shaft to displace drive racks, which displace wedge supports with restricted movement by limiting shafts, ultimately promoting the displacement of the wedge supports, which push retractable rollers by inserting them into the side cavities of the anchoring port preventing the disengagement thereof.

In the instrument, object of the invention, the perimeter holes are located forming two parallel circumferences in the cylindrical body of the ring.

In the instrument, object of the invention, the cylindrical body of the ring comprises a perimeter groove, configured to fix the vascular prosthesis by means of a removable fixation element.

In the instrument, object of the invention, the anchoring port, integrated in the ring, comprises a bushing for embracing the cylindrical body of the ring with rotary translation capacity around the cylindrical body and which is fixed to said cylindrical body by means of a clamping nut; in addition, it comprises an indexing wedge that, in combination with a toothed gear of the cylindrical body, performs the correct positioning of the head in the different stapling positions.

In the instrument, object of the invention, the indexing wedge comprises a spring, housed in its corresponding sleeve, where a fixing pin serves as a stop for the sleeve, limiting the compression of the spring.

In the instrument, object of the invention, the internal transmission means of the stem comprise a series of transmission cables and their corresponding housing conduits.

In the instrument, object of the invention, the head comprises a mechanism box that incorporates a drive coil of the anchoring mechanism, a drive coil of the compression-decompression mechanism and a drive coil of the firing mechanism, such that each of the coils is associated with the transmission cables of the stem, and activate the anchoring mechanism between the head and the ring, the compression-decompression mechanism and the firing mechanism of the anterolateral staples.

In the instrument, object of the invention, the body of the head has a curvature of the contact surface that is in line with the ring, respecting the morphology of the circumference of the aortic section.

In the instrument, object of the invention, the body of the head comprises grooves configured to accompany the ejection of the anterolateral staples the optimal distance for a correct B-shaped deformation.

### BRIEF DESCRIPTION OF THE DRAWINGS

To complement the description that will be made below and in order to help a better understanding of the features of the invention, the present specification is accompanied by a set of Figs., on the basis of which the innovations and advantages of the instrument for surgical stapling between a vascular prosthesis and the aorta object of the invention will be more easily understood.

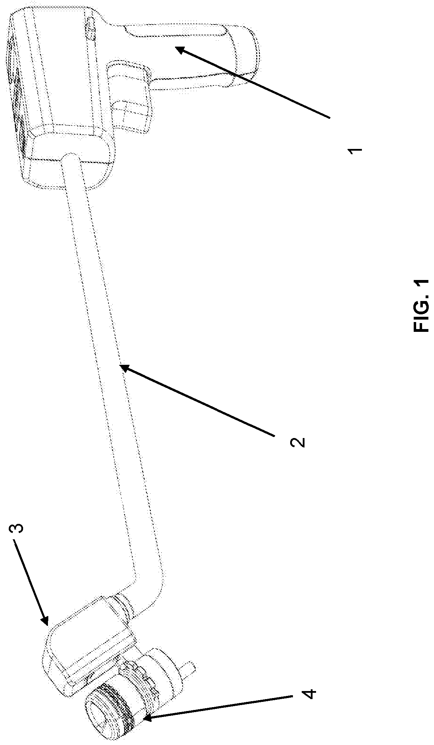

Fig. 1 shows a perspective view of the instrument for surgical stapling between a vascular prosthesis and the aorta object of the invention.

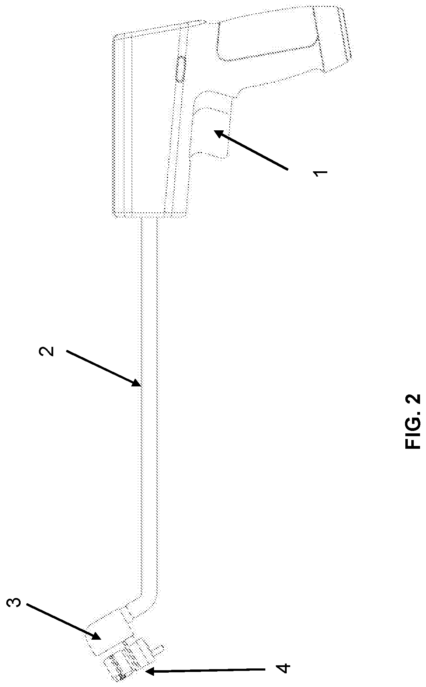

Fig. 2 shows a side view of the instrument for surgical stapling between a vascular prosthesis and the aorta object of the invention.

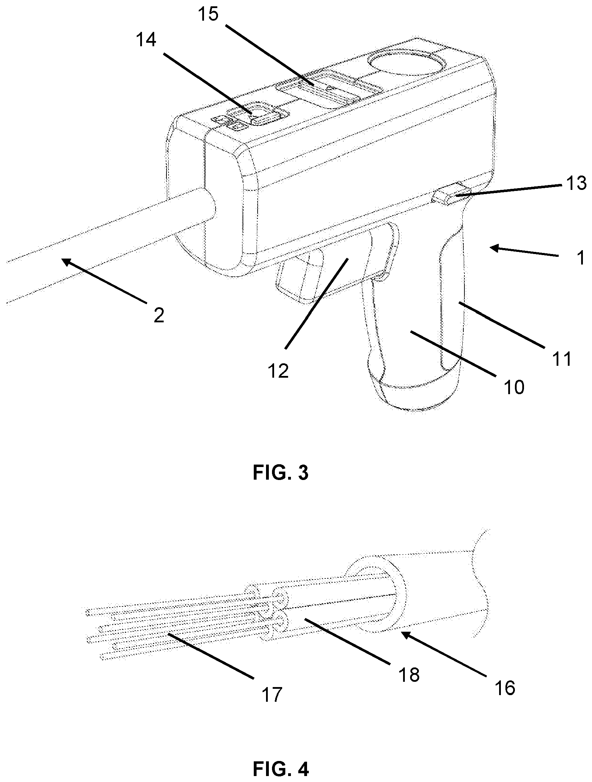

Fig. 3 shows a perspective view of the handle of the instrument for surgical stapling between a vascular prosthesis and the aorta object of the invention.
Fig. 4 shows a perspective view of the sectional stem of the instrument for surgical stapling between a vascular prosthesis and the aorta object of the invention.

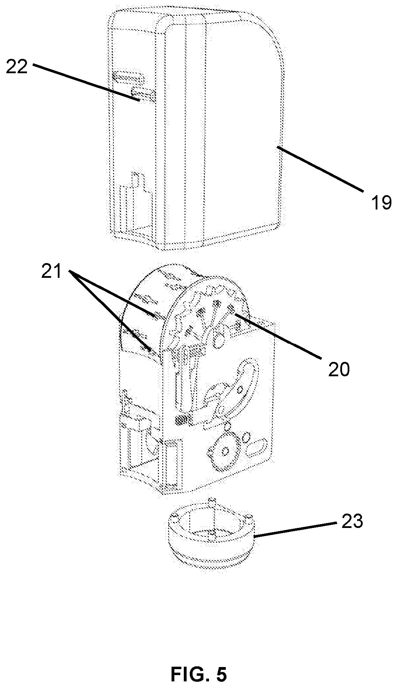

Fig. 5 shows a perspective view of the head of the instrument for surgical stapling between a vascular prosthesis and the aorta, object of the invention, partially exploded.

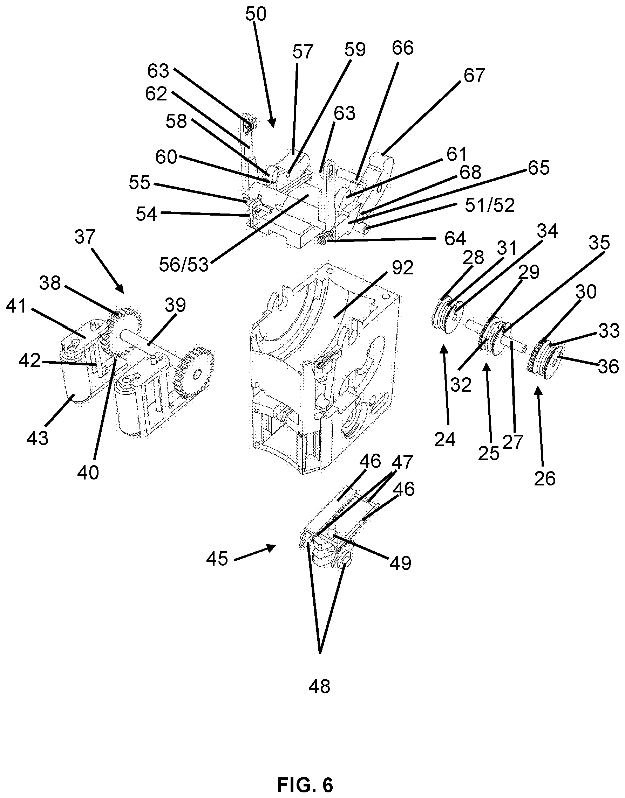

Fig. 6 shows an exploded perspective view of the mechanisms of the head of the instrument for surgical stapling between a vascular prosthesis and the aorta, object of the invention.
Fig. 7 shows a partially exploded perspective view of the rotary head having ejected the anterolateral staples.4
Fig. 8 shows a rotary head section with the anterolateral staples inside.
Fig. 9 shows a rotary head section with the anterolateral staples out.

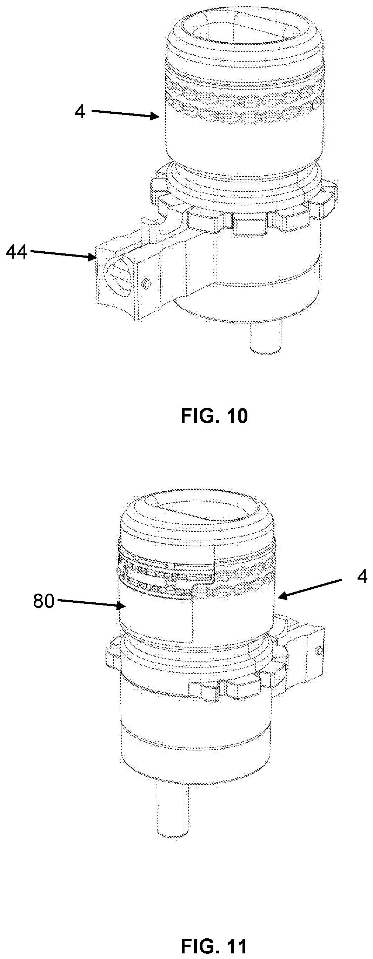

Fig. 10 shows a perspective view of the ring of the instrument, object of the invention.
Fig. 11 shows a perspective view of the ring of the instrument, object of the invention, other than that of Fig. 10.

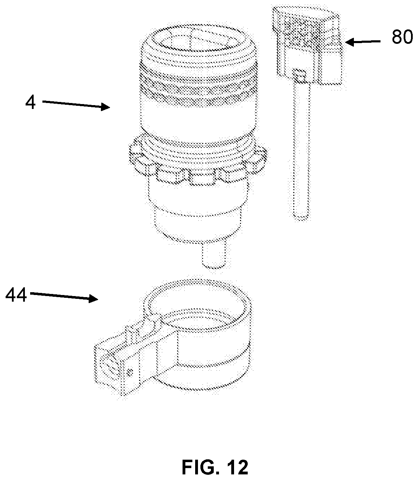

Fig. 12 shows a partially exploded perspective view of the ring of the instrument, object of the invention.

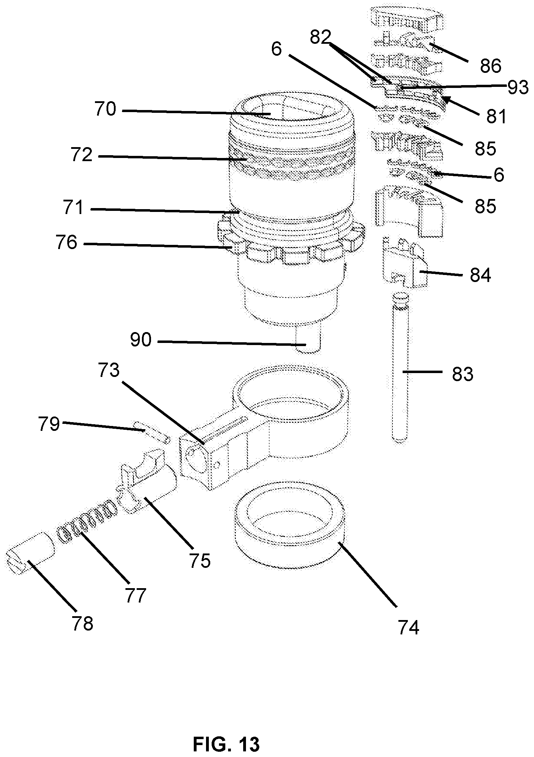

Fig. 13 shows an exploded perspective view of the ring of the instrument, object of the invention.

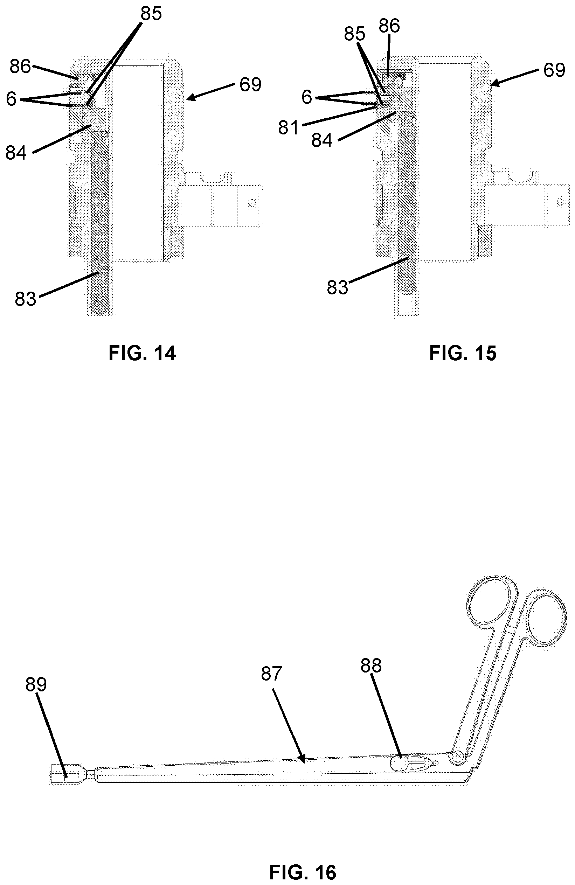

Fig. 14 shows a sectional view of the stapling mechanism for the posterior aortic sector with the posterior staples inside.
Fig. 15 shows a sectional view of the stapling mechanism for the posterior aortic sector with the posterior staples out.
Fig. 16 shows a view of the auxiliary grippers of the instrument, object of the invention.

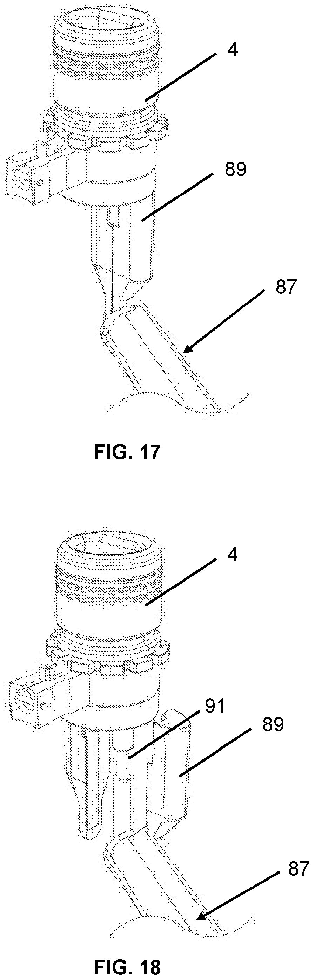

Fig. 17 shows the ring and auxiliary grippers assembly for viewing the attachment between these elements and the firing element of the stapling mechanism for the posterior aortic sector.
Fig. 18 shows the ring and auxiliary grippers assembly exploded to see the fixation between these elements and the firing element of the stapling mechanism for the posterior aortic sector.

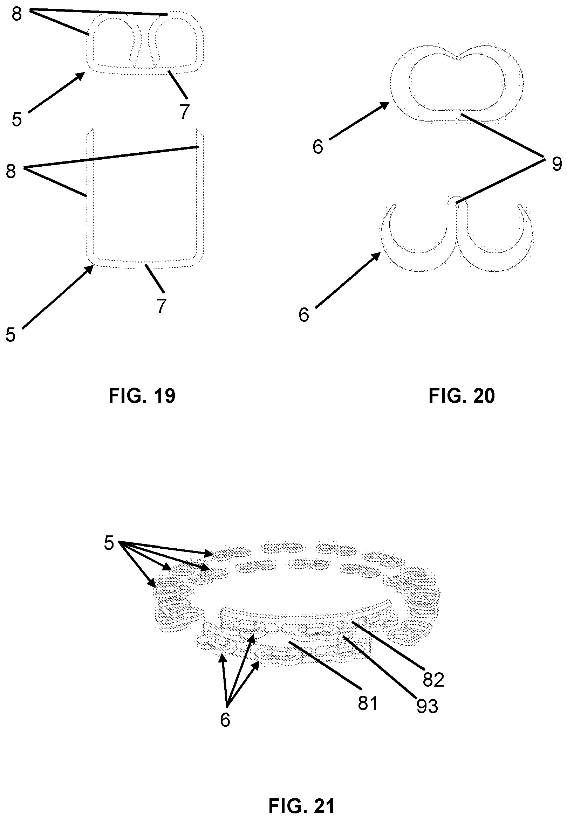

Fig. 19 shows two plan views of the anterolateral staples, one in before-use position and one in-use position, i.e. deformed into a B-shape
Fig. 20 shows two plan views of the posterior staples, one in before-use position and another in-use position.
Fig. 21 shows a perspective view of the two rows of stapling elements that make up a complete staple with the anterolateral staples, the posterior staples and the stapling sheet.

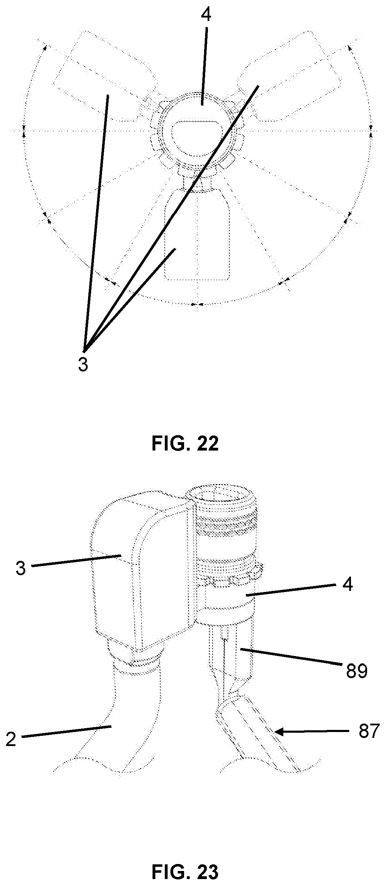

Fig. 22 shows a representation of the different positions of the head with respect to the ring for a complete stapling.
Fig. 23 shows a perspective view of a ring showing the auxiliary grippers placing the ring and the head fixed to the ring.

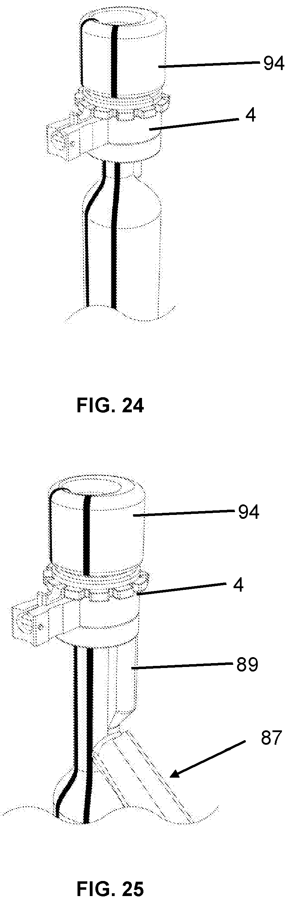

Fig. 24 shows a perspective view of a ring with the vascular prosthesis in the inner passage of the cylindrical body of the ring.
Fig. 25 shows a perspective view of a ring with the vascular prosthesis in the inner passage of the cylindrical body of the ring and the auxiliary grippers placing the ring.

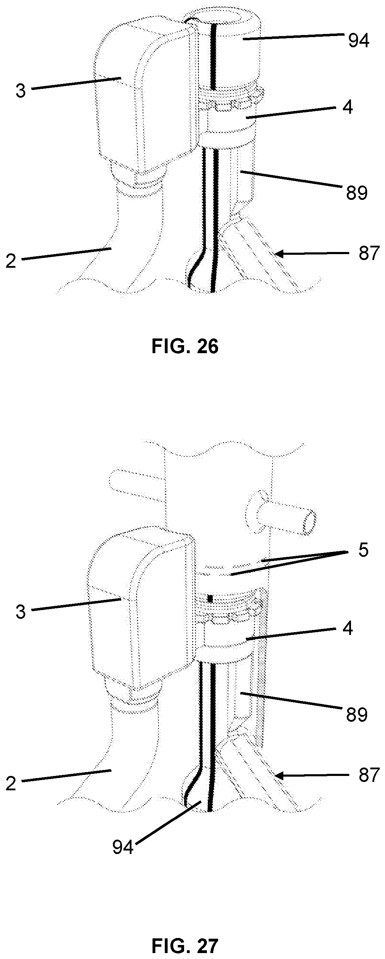

Fig. 26 shows a perspective view of a ring with the vascular prosthesis in the inner passage of the cylindrical body of the ring showing the auxiliary grippers placing the ring, the head fixed to the ring.
Fig. 27 shows a view like that of Fig. 26 showing the aorta to which the vascular prosthesis is attached with the anterolateral staples implemented.

The numerical references used in the Figs. are:
- 1.: handle,
- 2.: stem,
- 3.: head,
- 4.: ring,
- 5.: anterolateral staple
- 6.: posterior staple,
- 7.: crown,
- 8.: leg,
- 9.: central zone,
- 10.: ergonomic shell
- 11.: non-slip zone
- 12.: trigger,
- 13.: head position lock pushbutton,
- 14.: anchoring/release selector,
- 15.: emergency release button,
- 16.: spindle shaft,
- 17.: transmission cable,
- 18.: housing conduit,
- 19.: head body,
- 20.: rotary magazine,
- 21.: perimeter housing,
- 22.: groove,
- 23.: rotating joint,
- 24.: anchoring mechanism drive coil,
- 25.: compression-decompression mechanism drive coil,
- 26.: firing mechanism drive coil,
- 27.: rotation shaft,
- 28.: transmission pulley,
- 29.: transmission pulley,
- 30.: transmission pulley,
- 31.: transmission cable,
- 32.: transmission cable,
- 33.: transmission cable,
- 34.: fixation bolt,
- 35.: fixation bolt
- 36.: fixation bolt
- 37.: anchoring mechanism between the head and the ring,
- 38.: driven gears,
- 39.: solidary shaft,
- 40.: drive racks,
- 41.: wedge supports,
- 42.: limiting shafts,
- 43.: retractable rollers,
- 44.: anchoring port,
- 45.: compression - decompression mechanism,
- 46.: driven racks,
- 47.: joining columns,
- 48.: traction eccentrics,
- 49.: stop cam,
- 50.: firing mechanism,
- 51.: driven gear,
- 52.: shaft,
- 53.: runner,
- 54.: telescopic cam shaft,
- 55.: cam limiting shaft,
- 56.: coupling shaft,
- 57.: fork,
- 58.: drive pawl,
- 59.: shaft,
- 60.: recovery spring,
- 61.: fixer,
- 62.: firing levers,
- 63.: rack skids,
- 64.: spring,
- 65.: friction wedge,
- 66.: shaft,
- 67.: dynamic stop,
- 68.: oscillating pivot,
- 69.: cylindrical body of the ring,
- 70.: inner conduit,
- 71.: perimeter groove,
- 72.: perimeter holes,
- 73.: bushing,
- 74.: clamping nut,
- 75.: indexing wedge,
- 76.: toothed gear,
- 77.: spring,
- 78.: sleeve,
- 79.: fixing pin,
- 80.: stapling mechanism for the posterior aortic sector,
- 81.: stapling sheet,
- 82.: through holes of the stapling sheet for ejecting the posterior staples,
- 83.: actuating stem,
- 84.: sliding wedges,
- 85.: plates,
- 86.: retaining flaps,
- 87.: auxiliary gripper,
- 88.: sliding pull,
- 89.: adapter,
- 90.: clamping cannula,
- 91.: driving cylindrical element,
- 92.: mechanism box,
- 93.: through-holes in the stapling sheet for additional sutures,
- 94.: vascular prosthesis,
- 95.: geared levers, and
- 96.: sliding plates.

### DETAILED DESCRIPTION OF THE INVENTION

An object of the invention is to provide an instrument for surgical stapling between a vascular prosthesis (94) and the aorta.

The aim of stapling is the implementation, by means of successive firings, of two rows of overlapping stapling elements in a brick arrangement. This stapling establishes the permanent attachment between a vascular prosthesis (94) and the aortic tissue. In turn, the final distribution of the two rows of stapling elements in brick arrangement determines compliance with the principles of mechanical bonding objective of the intervention, that is tissue without tension; tightness of the attachment; absence of leaks; correct hemostasis; adequate perfusion of the anastomotic edges and adequate lumen.

The instrument for surgical stapling between a vascular prosthesis (94) and the aorta, object of the invention, comprises a handle (1), a stem (2), a head (3) and a ring (4), such that the handle (1) is configured to handle the head (3), and together with the ring (4) perform surgical stapling between a vascular prosthesis (94) and the aorta.

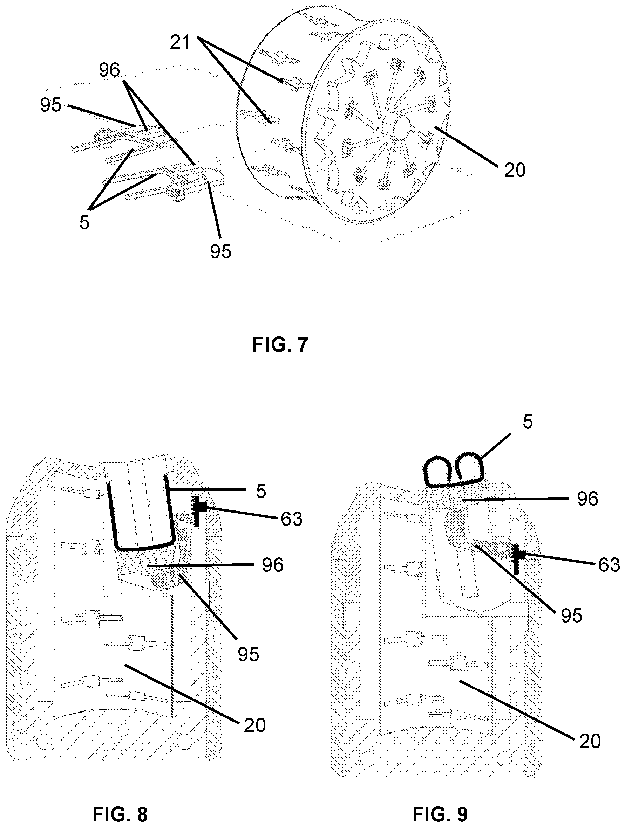

The instrument, object of the invention, employs stapling elements of two types for its purpose that are hereinafter referred to as anterolateral staples (5) and posterior staples (6), this denomination attending to the position of the stapling elements when the instrument is placed in its working position. The anterolateral staples (5) before use are U-shaped with two legs (7) and a crown (8), such that the legs (7), when the anterolateral staples (5) are used, deform until they acquire a B-shape (see Fig. 8). The other stapling elements are the posterior staples (6) which before use have a curved W-shape such that when used the central zone (9) of the W is pressed and deformed until the ends of the W contact providing a closed shape (see Fig. 9).

The crown (8) of the anterolateral staples (5) describes an arc of circumference in accordance with the diameter of the aorta being stapled, adapting itself correlatively to its shape and balancing the pressure exerted by each anterolateral staple (5).

The handle (1) comprises an ergonomic shell (10) that has a non-slip zone (11) and a trigger (12) configured to give two instructions, a first instruction to compress the tissues, with a first travel of the trigger (12), and a second instruction to expel the anterolateral staples (5) two by two; for this, the trigger (12) has a travel divided into two sections, a first section that marks the compression position of the tissues and a second section that marks the placement of the anterolateral staples (8).

The handle (1) also comprises a head position lock pushbutton (13) so that, with the pushbutton pressed, the head (3) can displace and, if it is no longer pressed, the head (3) is locked in one position. The handle (1) also comprises an anchoring/release selector (14) of the head (3) with respect to the ring (4) that allows the fixation between the head (3) and the ring (4) to be released. The handle (1) also comprises an emergency release button (15) that activates a decompression system, in the event that the surgeon observes any interference during stapling. This decompression system is activated by displacing the emergency release button (15) located on the upper face of the handle (1). This movement is performed forward in correlation with the return travel of the trigger (12) to its initial position. Said emergency release button (15) returns the trigger (12) to its initial position and decompresses the tissues if necessary. The return of the emergency release button (15) occurs automatically, so that it is always in a neutral position.

The stem (2) comprises a shaft (16) and internal transmission means configured to transfer the instructions given by the handle (1) to the head (3) for the different operations and functions of the handle (1).

The internal transmission means comprise a series of transmission cables (17) and their corresponding housing conduits (18). The transmission cables (17) connect the pushbuttons or devices integrated in the handle (1) with a series of associated mechanisms of the head (3), allowing both their activation and their return. The housing conduits (18) collect the transmission cables (17) along their path to avoid unwanted crossings between said elements, holding each of them independent of the others.

The head (3) comprises a body (19) where a rotary magazine (20) is housed which, in turn, has a plurality of perimeter housings (21), configured to house the anterolateral staples (5) before being placed in their working position for stapling and through which said anterolateral staples (5) come out when they are placed in their stapling position. The body (19) of the head (3) acts, in turn, as a compressive element and, therefore, has a curvature of the contact surface that is in line with the ring (4), respecting the morphology of the circumference of the aortic section, which favors the homogeneous compression of the tissues to be stapled. In turn, in the aforementioned contact surface you can see small grooves (22) whose purpose is to accompany the ejection of the anterolateral staples (5) the optimal distance for a correct B-shaped deformation.

In the preferred embodiment of the invention, the anterolateral staples (5) are ejected sequentially in pairs, for which the head (3) comprises a firing mechanism (50) of the anterolateral staples (5) in pairs. Likewise, the head (3) comprises means for providing the rotary movement to the rotary magazine (20).

The head (3) also comprises a rotating joint (23) that provides a rotating movable joint between the stem (2) and the head (3) itself. This movable joint allows an optimal displacement of the head (3) around the ring (4), to travel the appropriate distances between the different stapling positions, respecting the angles of incidence of the stapler with respect to the surgical field.

The head (3) comprises an anchoring mechanism drive coil (24), a compression-decompression mechanism drive coil (25) and a firing mechanism drive coil (26) incorporated in a mechanism box (92). Each of the coils (24,25,26) is associated with the transmission cables (17) of the stem (2), and promotes the activation of their corresponding linked mechanical systems, i.e. an anchoring mechanism between the head and the ring (37), a compression-decompression mechanism (45) and the firing mechanism (50) of the anterolateral staples (5).

The coils (24,25,26) are arranged attached to one another by a rotation shaft (27). In turn, each coil (24,25,26) comprises a transmission pulley (28,29,30) with a built-in gear, a transmission cable (31,32,33) screwed onto the pulley (28,29,30), and a fixation bolt (34, 35, 36) for fixation of the transmission cable (31,32,33).

The anchoring mechanism between the head and the ring (37), has the function of fixing the head (3) to the ring (4) by means of an anchoring port (44) comprised in the ring (4), to establish a stable union between said components, while the successive firings of the anterolateral staples (5) are carried out. Likewise, it allows the release of the head (3) with respect to the ring (4) once the stapling is finished.

The activation of the anchoring mechanism between the head and the ring (37) is determined by the anchoring-release selector (14) located on the handle (1), which, through transmission cables (17), which pull the transmission pulley (28) integrated in the assembly of the drive coil of the anchoring mechanism (24), promotes rotation in one direction or another to achieve the activation or deactivation of the anchoring mechanism between the head and the ring (37).

The anchoring mechanism between the head and the ring (37) comprises driven gears (38), associated with the gear of the transmission pulley (38) of the coil (24) and joined together by an integral shaft (39), which displace drive racks (40), which move wedge supports (41), whose movement is restricted by limiting shafts (42), ultimately promoting the displacement of the wedge supports (41), which push retractable rollers (43) introducing them into the lateral cavities of the anchoring port (44) and preventing the disengagement thereof. In turn, the retractable rollers (43) limit the movement of the head (3) on the anchoring port (44) during its compression/decompression and transfer phases.

The head (3) comprises a compression-decompression mechanism (45) whose function is to displace the body (19) to perform a homogeneous compression of the tissues to be stapled, prior to the firing of anterolateral staples (5) in a certain position.

The activation of the compression-decompression mechanism (45) is determined by the pulsation (up to its first travel) of the trigger (12) located on the handle (1), which through transmission cables (17) rotates the transmission pulley (29) of the drive coil of the compression-decompression mechanism (25), and promotes the activation of said compression-decompression mechanism (45). In turn, the activation of the emergency release button (15) located on the handle (1), allows the tissues to be decompressed, making a return movement of the compression-decompression mechanism (45).

The compression-decompression mechanism (45) comprises driven racks (46), associated with the gear of the transmission pulley (29) of the coil (25) and joined together by joining columns (47), which rotate traction cams (48), ultimately promoting a forward movement of the head (3) that exerts the optimal compression of the tissues to be stapled. In turn, there is a stop cam (49) that determines the limitation of the decompression movement.

The head (3) comprises a firing mechanism (50) of the anterolateral staples (5), the function of which is to actuate the rotary magazine (20) to perform the firing of two anterolateral staples (5) of alternating rows in a brick arrangement. Likewise, a return movement of the firing mechanism (50) occurs after each firing.

The activation of the firing mechanism (50) is determined by the pulsation (up to its second travel) of the trigger (12) located on the handle (1), which, through transmission cables (17), rotates the transmission pulley (30) of the drive coil of the firing mechanism (26), and promotes the activation of said firing mechanism (50).

This firing mechanism (50) comprises a driven gear (51), associated with the transmission pulley gear (30) of the coil (26) and mounted on a shaft (52), which displaces a runner (53), provided with a telescopic cam shaft (54) and a cam limiting shaft (55). This runner (53), through a coupling shaft (56), allows the rotation of a fork (57) that is associated with a drive pawl (58), mounted on a shaft (59) and provided with a return spring (60), which promotes the rotation of the rotary magazine (20), which is positioned and prepared for the subsequent firing of the anterolateral staples (5). In the last section of the travel of the runner (53), once the rotation has been completed and the correct positioning of the rotary magazine (20) has been ensured by means of a fixer (61), the telescopic cam shaft (54) abuts the runner (53) itself, and is dragged along with it until said telescopic cam shaft (54) exerts the necessary pressure, ultimately promoting the activation of firing levers (62) that displace rack skids (63). These slides displace the geared levers (95) displacing the sliding plates (96) that eject the anterolateral staples (5) for their B-shaped deformation. In turn, a complementary mechanism allows the unlocking, dynamic travel stop and locking movements. Said complementary mechanism comprises a spring (64), which displaces a friction wedge (65), associated by means of a shaft (66) to a dynamic stop (67), which is attached by means of an oscillating pivot (68) to the fixer (61) that determines the position of the mechanical system by arranging it for firing.

Once the firing has been carried out, the mechanism is released and returns to its initial position by the effect of the transmission cables (17), which are pulled from the handle (1) in the opposite direction, and cause the reverse rotation of the transmission pulley (30), which returns said firing mechanism (50) to the neutral or previous position. During this return movement the rotating drum (20) is held still by both the effect of the fixer (61) and the tooth jump of the drive pawl (58).

The ring (4) comprises a cylindrical body (69) with an inner conduit (70) for housing the vascular prosthesis (94) therein and performing an eversion of the proximal end of said vascular prosthesis (94) on the cylindrical body (69). The cylindrical body (69) comprises a perimeter groove (71) configured to fix the vascular prosthesis (94) and ensure its correct position by means of a removable retaining element. The ring (4) comprises a plurality of perimeter holes (72) located forming two parallel circles, such that these perimeter holes (72) are configured to receive the legs (8) of the anterolateral staples (5) and provide them with the B-shape.

The ring (4) also comprises an anchoring port (44) for anchoring to the head (3), such that the anchoring port (44) comprises a bushing (73) that embraces the cylindrical body (69) of the ring (4), allowing its rotational translation around said element, and that is fixed thereto by means of a clamping nut (74); it also comprises an indexing wedge (75) that, in combination with a toothed gear (76) of the cylindrical body (69), allows the correct positioning of the head (3) in the different stapling positions. The indexing wedge (75) is provided with a spring (77), housed in its corresponding sleeve (78), which guarantees the correct damping of the system to describe the different positions of the head (3); in addition, a fixing pin (79) serves as a stop for the sleeve (78), limiting the adequate compression of the spring (77).

For stapling the part of the vascular prosthesis (94) that coincides with the spine, the ring (4) comprises a stapling mechanism for the posterior aortic sector (80) that includes a stapling sheet (81), the posterior staples (6) and a means for ejecting the posterior staples (6). The stapling sheet (81) describes an arc of circumference in accordance with the diameter of the vascular prosthesis (94) and the diameter of the aorta, and has a plurality of through holes (82). The posterior staples (6), before use, are located inside the ring (4). The stapling mechanism for the posterior aortic sector (80) works as follows: the means for ejecting the posterior staples (6) push the posterior staples (6) towards the outside of the ring (4) until they contact the stapling sheet (81) through the central zone (9), this causes them to deform acquiring the closed shape that compresses the vascular prosthesis (94) and the wall of the aorta located on the stapling sheet (81) between its limits, thus fixing the vascular prosthesis (94) to the aorta through the stapling sheet (81) and the posterior staples (6).

The means for ejecting the posterior staples comprise an actuating stem (83), which displaces a series of sliding wedges (84) (joined at their base for joint movement), which drive a series of flanges (85) that eject the posterior staples (6) for implementation. In its last stretch of travel, the sliding wedges (84) are associated with retaining flaps (86), which describe a backward movement that releases the stapling sheet (81) from its fixation with respect to the stapling mechanism for the posterior aortic sector (80).

The stapling sheet (81) comprises two through holes (93) intended for the possible incorporation of additional manual sutures if necessary.

For the introduction and extraction of the ring (4) in the surgical field of the intervention, in addition to its fixation in the aortic stapling section, the instrument, object of the invention, comprises an auxiliary gripper (87) which, in addition to serving for the placement of the ring (4) in the aforementioned position of use, comprises a sliding pull (88) for actuating the stapling mechanism for the posterior aortic sector (80) incorporated in the ring (4).

For a correct fixation with the cylindrical body (69) of the ring (4), the auxiliary gripper (87) has at its distal end an adapter (89) that surrounds a fastening cannula (90) located in the ring (4) itself. In addition, the adapter (89) contains a cylindrical pusher element (91) that activates the stapling mechanism for the posterior aortic sector (80) incorporated in the ring (4).

## Claims

1. Instrument for surgical stapling between a vascular prosthesis (94) and the aorta, wherein it comprises:
- a handle (1),
- a stem (2) associated with the handle (1),
- a head (3) associated with the stem,
- a ring (4) associated with the head,
- anterolateral staples (5),
- posterior staples (6),
wherein the handle (1) comprises:
- an ergonomic shell (10) with a trigger (12) configured to give two instructions, a first tissue compression instruction, with a first travel of the trigger (12), and a second ejection instruction of the anterolateral staples (5) two by two, with a second travel of the trigger (12);
- a head position lock pushbutton (13) for locking the head (3) in a position relative to the ring (4);
- an anchoring/release selector (14) for fixing or releasing the head (3) with respect to the ring (4); and
- an emergency release button (15) for activating a decompression system;
wherein the stem (2) comprises:
- a shaft (16) and
- internal transmission means for transferring the instructions given by the handle (1) to the head (3),
wherein the head (3) comprises:
- a body (19) where a rotary magazine (20) is housed which, in turn, has a plurality of perimeter housings (21), configured to house the anterolateral staples (5) before being placed in their working position for stapling and through which the aforementioned anterolateral staples (5) come out when they are placed in their stapling position;
- an anchoring mechanism between the head and the ring (37) for fixing the head (3) to the ring (4);
- a compression-decompression mechanism (45) to displace the body (19) and perform a homogeneous compression of the tissues to be stapled, prior to the firing of anterolateral staples (5);
- a firing mechanism (50) of the anterolateral staples (5) to actuate the rotary magazine (20) to perform the firing of two anterolateral staples (5);
- means for providing a turning movement to the rotary magazine;
- a rotating joint (23) to provide a rotating movable joint between the stem (2) and the head (3) itself; and
wherein the ring (4) comprises:
- a cylindrical body (69) with an inner conduit (70) for housing the vascular prosthesis (94) therein and overlapping the distal end of said vascular prosthesis (94) on the cylindrical body (69).
- a plurality of perimeter holes (72) configured to receive the legs (6) of the anterolateral staples (5) and provide them with the B-shape;
- an anchoring port (44) for its fixation and release with respect to the head (3);
- a stapling mechanism for the posterior aortic sector (80) comprising a stapling sheet (81) with a plurality of through holes (82), the posterior staples (6) and a posterior staple ejection means (6), where the posterior staple ejection means (6) pushes the posterior staples (6) towards the outside of the ring (4) until they contact the stapling sheet (81) through the central zone (9), and this causes them to deform acquiring the closed shape that compresses the vascular prosthesis (94) and the wall of the aorta located on the stapling sheet (81) between its limits, thus fixing the vascular prosthesis (94) to the aorta through the stapling sheet (81) and the posterior staples (6).

2. Instrument for surgical stapling between a vascular prosthesis (94) and the aorta, according to claim 1, **characterized in that** the means for ejecting the posterior staples comprise an actuator stem (83) to displace a series of sliding wedges (84) to drive a series of plates (85) that eject the posterior staples (6) where the sliding wedges (84) are associated with retaining flaps (86), which describe a backward movement to release the stapling sheet (81) from its fixation with respect to the stapling mechanism for the posterior aortic sector (80).

3. Instrument for surgical stapling between a vascular prosthesis (94) and the aorta, according to any one of claims 1 to 2, **characterized in that** it comprises an auxiliary gripper (87) comprising a pull (88) for actuating the stapling mechanism for the posterior aortic sector (80) incorporated in the ring (4).

4. Instrument for surgical stapling between a vascular prosthesis (94) and the aorta, according to claim 3 **characterized in that** the auxiliary gripper (87) comprises an adapter (89) at its distal end that surrounds a fastening cannula (90) located in the ring (4) itself, such that the adapter (89) contains a driving cylindrical element (91) that activates the stapling mechanism for the posterior aortic sector (80) incorporated in the ring (4).

5. Instrument for surgical stapling between a vascular prosthesis (94) and the aorta, according to any one of claims 1 to 4, **characterized in that** the stapling sheet (81) comprises two through holes (93) configured to incorporate additional sutures.

6. Instrument for surgical stapling between a vascular prosthesis (94) and the aorta, according to any of claims 1 to 5, **characterized in that** the firing mechanism (50) comprises a driven gear (51) mounted on a shaft (52), which displaces a runner (53), provided with a telescopic cam shaft (54) and a limiting cam shaft (55), such that the runner (53), through a coupling shaft (56), allows the rotation of a fork (57) that is associated with a drive pawl (58), mounted on a shaft (59) and provided with a return spring (60), which promotes the rotation of the rotary magazine (20), such that the runner (53) has a last section where once the rotation is completed and the correct positioning of the rotary magazine (20) is ensured by means of a fixer (61), the telescopic cam shaft (54) abuts the runner (53) itself, and is dragged along with it until said telescopic cam shaft (54) exerts the necessary pressure, ultimately promoting the activation of firing levers (62) that displace rack skids (63), such that these rack skids (63) displace geared levers (95) that displace sliding plates (96) that eject the anterolateral staples (5) for their B-shaped deformation.

7. Instrument for surgical stapling between a vascular prosthesis (94) and the aorta, according to claim 6, **characterized in that** it comprises a complementary mechanism comprising a spring (64) configured to displace a friction wedge (65), associated by a shaft (66) to a dynamic stop (67), which is attached by means of an oscillating pivot (68) to the fixer (61) that determines the position of the mechanical system to allow unlocking, dynamic stop travel and locking movements.

8. Instrument for surgical stapling between a vascular prosthesis (94) and the aorta, according to any of claims 1 to 7, **characterized in that** the compression-decompression mechanism (45) comprises guided racks (46) joined together by joining columns (47) configured to rotate traction eccentrics (48), providing an advancing movement of the head (3) to exert the compression of the tissues to be stapled, it also comprises a stop cam (49) to limit the decompression movement.

9. Instrument for surgical stapling between a vascular prosthesis (94) and the aorta, according to any of claims 1 to 8, **characterized in that** the anchoring mechanism between the head and the ring (37) comprises driven gears (38) joined together by an integral shaft (39) to displace drive racks (40), which displace wedge supports (41) with restricted movement by limiting shafts (42), ultimately promoting the displacement of the wedge supports (41), which push retractable rollers (43) introducing them into the lateral cavities of the anchoring port (44) and preventing the disengagement thereof.

10. Instrument for surgical stapling between a vascular prosthesis (94) and the aorta, according to any one of claims 1 to 9, **characterized in that** the perimeter holes (72) are located forming two parallel circumferences in the cylindrical body (69) of the ring (4).

11. Instrument for surgical stapling between a vascular prosthesis (94) and the aorta according to any one of claims 1 to 10, **characterized in that** the cylindrical body (69) comprises a perimeter groove (71) configured to fix the vascular prosthesis (94) by means of a removable fixation element.

12. Instrument for surgical stapling between a vascular prosthesis (94) and the aorta according to any of claims 1 to 11, **characterized in that** the anchoring port (44) comprises a bushing (73) for embracing the cylindrical body (69) of the ring (4) with rotating translation capacity around the cylindrical body (69) and which is fixed to said cylindrical body (69) by means of a clamping nut (74); it also comprises an indexing wedge (75) which, in combination with a toothed gear (76) of the cylindrical body (69), performs the correct positioning of the head (3) in the different stapling positions.

13. Instrument for surgical stapling between a vascular prosthesis (94) and the aorta according to claim 12, **characterized in that** the indexing wedge (75) comprises a spring (77), housed in its corresponding sleeve (78), where a fixing pin (79) serves as a stop for the sleeve (78), limiting the compression of the spring (77).

14. Instrument for surgical stapling between a vascular prosthesis (94) and the aorta according to any one of claims 1 to 13, **characterized in that** the internal transmission means of the stem comprise a series of transmission cables (17) and their corresponding housing conduits (18).

15. Instrument for surgical stapling between a vascular prosthesis (94) and the aorta according to claim 14 **characterized in that** the head (3) comprises a mechanism box (92) that incorporates an actuation coil of the anchoring mechanism (24), an actuation coil of the compression-decompression mechanism (25) and an actuation coil of the firing mechanism (26), such that each of the coils (24,25,26) is associated with the transmission cables (17) of the stem (2), and activate the anchoring mechanism between the head and the ring (37), the compression-decompression mechanism (45) and the firing mechanism (50) of the anterolateral staples (5).

16. Instrument for surgical stapling between a vascular prosthesis (94) and the aorta according to any one of claims 1 to 15, **characterized in that** the body (19) of the head (3) has a curvature of the contact surface that is in line with the ring (4) respecting the morphology of the circumference of the aortic section.

17. Instrument for surgical stapling between a vascular prosthesis (94) and the aorta according to any one of claims 1 to 16, **characterized in that** the body (19) of the head (3) comprises grooves (22) configured to accompany the ejection of the anterolateral staples (5) the optimal distance for a correct B-shaped deformation.

## Patentansprüche

1. Chirurgisches Klammerinstrument zwischen einer Gefäßprothese (94) und der Aorta,
wobei es Folgendes umfasst:
- einen Griff (1),
- einen Schaft (2), dem der Griff (1) zugeordnet ist,
- einen Kopf (3), dem der Schaft zugeordnet ist,
- einen Ring (4), dem der Kopf zugeordnet ist,
- anterolaterale Klammern (5),
- hintere Klammern (6),
wobei der Griff (1) Folgendes umfasst:
- eine ergonomische Schale (10) mit einem Auslöser (12), der dazu konfiguriert ist, zwei Anweisungen zu geben, eine erste Gewebekompressionsanweisung mit einem ersten Hub des Auslösers (12) und eine zweite Ausstoßanweisung der anterolateralen Klammern (5) paarweise mit einem zweiten Hub des Auslösers (12);
- einen Druckknopf (13) zum Arretieren der Kopfposition, um den Kopf (3) in einer Position relativ zum Ring (4) zu arretieren;
- einen Verankerungs-/Freigabeselektor (14) zum Fixieren oder Freigeben des Kopfes (3) in Bezug auf den Ring (4); und
- einen Notfreigabeknopf (15) zum Aktivieren eines Dekompressionssystems;
wobei der Schaft (2) Folgendes umfasst:
- eine Welle (16) und
- interne Übertragungsmittel zum Weiterleiten der vom Griff (1) gegebenen Anweisungen an den Kopf (3),
wobei der Kopf (3) Folgendes umfasst:
- einen Körper (19), in dem ein Drehmagazin (20) aufgenommen ist, das wiederum eine Vielzahl von Umfangsgehäusen (21) aufweist, die dazu konfiguriert sind, die anterolateralen Klammern (5) aufzunehmen, bevor sie in ihre Arbeitsposition zum Klammern platziert werden, und durch die die vorgenannten anterolateralen Klammern (5) austreten, wenn sie in ihre Klammerposition platziert werden;
- einen Verankerungsmechanismus zwischen dem Kopf und dem Ring (37) zum Fixieren des Kopfes (3) am Ring (4);
- einen Kompressions-Dekompressionsmechanismus (45) zum Verschieben des Körpers (19) und zum Durchführen eines homogenen Zusammendrückens der zu klammernden Gewebe vor dem Austreiben der anterolateralen Klammern (5);
- einen Austreibungsmechanismus (50) für die anterolateralen Klammern (5), um das Drehmagazin (20) zu betätigen und das Austreiben von zwei anterolateralen Klammern (5) durchzuführen;
- Mittel, um eine Drehbewegung für das Drehmagazin bereitzustellen;
- ein Drehgelenk (23), um ein drehbares bewegliches Gelenk zwischen dem Schaft (2) und dem Kopf (3) selbst bereitzustellen; und
wobei der Ring (4) Folgendes umfasst:
- einen zylindrischen Körper (69) mit einem inneren Kanal (70) zum Aufnehmen der Gefäßprothese (94) darin und zum Überlappen des distalen Endes der Gefäßprothese (94) auf dem zylindrischen Körper (69).
- eine Vielzahl von Umfangsbohrungen (72), die dazu konfiguriert sind, die Schenkel (6) der anterolateralen Klammern (5) aufzunehmen und ihnen die B-Form zu verleihen;
einen Verankerungsanschluss (44) für seine Fixierung und Freigabe in Bezug auf den Kopf (3);
- einen Klammermechanismus für den hinteren Aortensektor (80), umfassend eine Klammerplatte (81) mit einer Vielzahl von Durchgangslöchern (82), die hinteren Klammern (6) und ein hinteres Klammeraustoßmittel (6), wobei das hintere Klammeraustoßmittel (6) die hinteren Klammern (6) zur Außenseite des Rings (4) drückt, bis sie mit der Klammerplatte (81) über die zentrale Zone (9) in Kontakt kommen, wodurch sie sich verformen und eine geschlossene Form annehmen, die die Gefäßprothese (94) und die Wand der Aorta, die sich auf dem Klammerblatt (81) zwischen dessen Begrenzungen befindet, komprimiert, wodurch die Gefäßprothese (94) über die Klammerplatte (81) und die hinteren Klammern (6) an der Aorta fixiert wird.

2. Chirurgisches Klammerinstrument zwischen einer Gefäßprothese (94) und der Aorta nach Anspruch 1, **dadurch gekennzeichnet, dass** die Mittel zum Ausstoßen der hinteren Klammern einen Betätigungsschaft (83) umfassen, um eine Reihe von Gleitkeilen (84) zu verschieben, um eine Reihe von Platten (85) anzutreiben, die die hinteren Klammern (6) ausstoßen, wobei die Gleitkeile (84) Halteklappen (86) zugeordnet sind, die eine Rückwärtsbewegung beschreiben, um die Klammerplatte (81) aus ihrer Fixierung in Bezug auf den Klammermechanismus für den hinteren Aortensektor (80) zu lösen.

3. Chirurgisches Klammerinstrument zwischen einer Gefäßprothese (94) und der Aorta nach einem der Ansprüche 1 bis 2, **dadurch gekennzeichnet, dass** es einen Hilfsgreifer (87) umfasst, der ein Zugelement (88) zum Betätigen des Klammermechanismus für den hinteren Aortensektor (80) umfasst, der in den Ring (4) eingebaut ist.

4. Chirurgisches Klammerinstrument zwischen einer Gefäßprothese (94) und der Aorta nach Anspruch 3, **dadurch gekennzeichnet, dass** der Hilfsgreifer (87) einen Adapter (89) an seinem distalen Ende umfasst, der eine Befestigungskannüle (90) umgibt, die sich im Ring (4) selbst befindet, sodass der Adapter (89) ein zylindrisches Antriebselement (91) enthält, das den im Ring (4) eingebauten Klammermechanismus für den hinteren Aortensektor (80) aktiviert.

5. Chirurgisches Klammerinstrument zwischen einer Gefäßprothese (94) und der Aorta nach einem der Ansprüche 1 bis 4, **dadurch gekennzeichnet, dass** die Klammerplatte (81) zwei Durchgangslöcher (93) umfasst, die dazu konfiguriert sind, zusätzliche Nähte zu integrieren.

6. Chirurgisches Klammerinstrument zwischen einer Gefäßprothese (94) und der Aorta nach einem der Ansprüche 1 bis 5, **dadurch gekennzeichnet, dass** der Austreibungsmechanismus (50) ein angetriebenes Zahnrad (51) umfasst, das auf einer Welle (52) montiert ist und einen Läufer (53) verschiebt, der mit einer Teleskopnockenwelle (54) und einer Begrenzungsnockenwelle (55) versehen ist, sodass der Läufer (53) über eine Kupplungswelle (56) die Drehung einer Gabel (57) ermöglicht, die einer Antriebsklaue (58) zugeordnet ist, die auf einer Welle (59) montiert und mit einer Rückstellfeder (60) versehen ist, die die Drehung des Drehmagazins (20) fördert, sodass der Läufer (53) einen letzten Abschnitt aufweist, in dem nach Abschluss der Drehung und Sicherstellung der korrekten Positionierung des Drehmagazins (20) mittels einer Fixierung (61), die Teleskopnockenwelle (54) an dem Läufer (53) selbst anliegt und mit diesem mitgezogen wird, bis die Teleskopnockenwelle (54) den erforderlichen Druck ausübt, wobei letztendlich die Betätigung von Austreibungshebeln (62) gefördert wird, die Zahnstangenführungen (63) verschieben, sodass diese Zahnstangenführungen (63) die Getriebehebel (95) verschieben, die die Gleitplatten (96) verschieben, die die anterolateralen Klammern (5) für ihre B-förmige Verformung ausstoßen.

7. Chirurgisches Klammerinstrument zwischen einer Gefäßprothese (94) und der Aorta nach Anspruch 6, **dadurch gekennzeichnet, dass** es einen komplementären Mechanismus umfasst, der eine Feder (64) umfasst, die dazu konfiguriert ist, einen Reibkeil (65) zu verschieben, der über eine Welle (66) einem dynamischen Anschlag (67) zugeordnet ist, der mittels einer Schwenkachse (68) an der Fixierung (61) angebracht ist, die die Position des mechanischen Systems bestimmt, um Bewegungen zum Entsperren, zum dynamischen Anschlag und zum Arretieren zu ermöglichen.

8. Chirurgisches Klammerinstrument zwischen einer Gefäßprothese (94) und der Aorta nach einem der Ansprüche 1 bis 7, **dadurch gekennzeichnet, dass** der Kompressions-Dekompressionsmechanismus (45) geführte Zahnstangen (46) umfasst, die durch Verbindungssäulen (47) zusammengefügt sind, die dazu konfiguriert sind, Traktions-Exzenter (48) zu drehen, wobei eine Vorwärtsbewegung des Kopfes (3) bereitgestellt wird, um die Kompression der zu klammernden Gewebe auszuüben, wobei er auch eine Anschlagnocke (49) umfasst, um die Dekompressionsbewegung zu begrenzen.

9. Chirurgisches Klammerinstrument zwischen einer Gefäßprothese (94) und der Aorta nach einem der Ansprüche 1 bis 8, **dadurch gekennzeichnet, dass** der Verankerungsmechanismus zwischen dem Kopf und dem Ring (37) angetriebene Zahnräder (38) umfasst, die durch eine integrierte Welle (39) miteinander verbunden sind, um Antriebszahnstangen (40) zu verschieben, die Keilstützen (41) mit eingeschränkter Bewegung durch Begrenzungswellen (42) verschieben, was letztendlich die Verschiebung der Keilstützen (41) fördert, die einziehbare Rollen (43) drücken, wobei diese in die seitlichen Hohlräume des Verankerungsanschlusses (44) eingeführt werden und deren Lösen verhindern.

10. Chirurgisches Klammerinstrument zwischen einer Gefäßprothese (94) und der Aorta nach einem der Ansprüche 1 bis 9, **dadurch gekennzeichnet, dass** sich die Umfangsbohrungen (72) so befinden, dass sie zwei parallele Kreise im zylindrischen Körper (69) des Rings (4) bilden.

11. Chirurgisches Klammerinstrument zwischen einer Gefäßprothese (94) und der Aorta nach einem der Ansprüche 1 bis 10, **dadurch gekennzeichnet, dass** der zylindrische Körper (69) eine Umfangsnut (71) umfasst, die dazu konfiguriert ist, die Gefäßprothese (94) mittels eines entfernbaren Fixierungselements zu fixieren.

12. Chirurgisches Klammerinstrument zwischen einer Gefäßprothese (94) und der Aorta nach einem der Ansprüche 1 bis 11, **dadurch gekennzeichnet, dass** der Verankerungsanschluss (44) eine Buchse (73) umfasst, um den zylindrischen Körper (69) des Rings (4) mit Dreh- und Verschiebungsfähigkeit um den zylindrischen Körper (69) herum zu erfassen, und die mittels einer Klemmmutter (74) an dem zylindrischen Körper (69) fixiert ist; zudem umfasst er einen Indexierungskeil (75), der in Kombination mit einem gezahnten Zahnrad (76) des zylindrischen Körpers (69) die korrekte Positionierung des Kopfes (3) in den unterschiedlichen Klammerpositionen bewirkt.

13. Chirurgisches Klammerinstrument zwischen einer Gefäßprothese (94) und der Aorta nach Anspruch 12, **dadurch gekennzeichnet, dass** der Indexierungskeil (75) eine Feder (77) umfasst, die in ihrer entsprechenden Hülse (78) aufgenommen ist, wobei ein Fixierstift (79) als Anschlag für die Hülse (78) dient und die Kompression der Feder (77) begrenzt.

14. Chirurgisches Klammerinstrument zwischen einer Gefäßprothese (94) und der Aorta nach einem der Ansprüche 1 bis 13, **dadurch gekennzeichnet, dass** die internen Übertragungsmittel des Schafts eine Reihe von Übertragungskabeln (17) und deren entsprechende Gehäusekanäle (18) umfassen.

15. Chirurgisches Klammerinstrument zwischen einer Gefäßprothese (94) und der Aorta nach Anspruch 14, **dadurch gekennzeichnet, dass** der Kopf (3) eine Mechanismusbox (92) umfasst, die eine Betätigungsspule des Verankerungsmechanismus (24), eine Betätigungsspule des Kompressions-Dekompressionsmechanismus (25) und eine Betätigungsspule des Austreibungsmechanismus (26) enthält, sodass jede der Spulen (24, 25, 26) den Übertragungskabeln (17) des Schafts (2) zugeordnet ist und den Verankerungsmechanismus zwischen dem Kopf und dem Ring (37), den Kompressions-Dekompressionsmechanismus (45) und den Austreibungsmechanismus (50) der anterolateralen Klammern (5) aktiviert.

16. Chirurgisches Klammerinstrument zwischen einer Gefäßprothese (94) und der Aorta nach einem der Ansprüche 1 bis 15, **dadurch gekennzeichnet, dass** der Körper (19) des Kopfes (3) eine Krümmung der Kontaktfläche aufweist, die mit dem Ring (4) übereinstimmt und die Morphologie des Umfangs des Aortenabschnitts berücksichtigt.

17. Chirurgisches Klammerinstrument zwischen einer Gefäßprothese (94) und der Aorta nach einem der Ansprüche 1 bis 16, **dadurch gekennzeichnet, dass** der Körper (19) des Kopfes (3) Nuten (22) umfasst, die dazu konfiguriert sind, den Ausstoß der anterolateralen Klammern (5) über die optimale Entfernung für eine korrekte B-förmige Verformung zu begleiten.

## Revendications

1. Instrument pour l'agrafage chirurgical entre une prothèse vasculaire (94) et l'aorte,
dans lequel il comprend :
- une poignée (1),
- une tige (2) associée à la poignée (1),
- une tête (3) associée à la tige,
- un anneau (4) associé à la tête,
- des agrafes antérolatérales (5),
- des agrafes postérieures (6),
dans lequel la poignée (1) comprend :
- une coque ergonomique (10) avec une gâchette (12) configurée pour donner deux instructions, une première instruction de compression des tissus, avec une première course de la gâchette (12), et une seconde instruction d'éjection des agrafes antérolatérales (5) deux par deux, avec une seconde course de la gâchette (12) ;
- un bouton-poussoir de verrouillage de position de la tête (13) pour verrouiller la tête (3) dans une position par rapport à l'anneau (4) ;
- un sélecteur d'ancrage/libération (14) pour fixer ou libérer la tête (3) par rapport à l'anneau (4) ; et
- un bouton de libération d'urgence (15) pour activer un système de décompression ;
dans lequel la tige (2) comprend :
- un arbre (16) et
- des moyens internes de transmission pour transférer les instructions données par la poignée (1) à la tête (3),
dans lequel la tête (3) comprend :
- un corps (19) dans lequel est logé un magasin rotatif (20) qui, à son tour, présente une pluralité de logements périphériques (21), configurés pour loger les agrafes antérolatérales (5) avant leur mise en position de travail pour l'agrafage et par lesquels lesdites agrafes antérolatérales (5) sortent lorsqu'elles sont placées dans leur position d'agrafage ;
- un mécanisme d'ancrage entre la tête et l'anneau (37) pour fixer la tête (3) à l'anneau (4) ;
- un mécanisme de compression-décompression (45) pour déplacer le corps (19) et effectuer une compression homogène des tissus à agrafer, préalablement au tir des agrafes antérolatérales (5) ;
- un mécanisme de tir (50) des agrafes antérolatérales (5) pour actionner le magasin rotatif (20) afin d'effectuer le tir de deux agrafes antérolatérales (5) ;
- des moyens pour fournir un mouvement de rotation au magasin rotatif ;
- une articulation rotative (23) pour fournir une articulation mobile rotative entre la tige (2) et la tête (3) elle-même ; et
dans lequel l'anneau (4) comprend :
- un corps cylindrique (69) avec un conduit interne (70) pour loger la prothèse vasculaire (94) à l'intérieur et superposer l'extrémité distale de ladite prothèse vasculaire (94) sur le corps cylindrique (69) ;
- une pluralité de trous périphériques (72) configurés pour recevoir les pattes (6) des agrafes antérolatérales (5) et leur conférer la forme en B ;
- un port d'ancrage (44) pour sa fixation et sa libération par rapport à la tête (3) ;
- un mécanisme d'agrafage pour le secteur aortique postérieur (80) comprenant une feuille d'agrafage (81) avec une pluralité de trous traversants (82), les agrafes postérieures (6) et un moyen d'éjection des agrafes postérieures (6), dans lequel le moyen d'éjection des agrafes postérieures (6) pousse les agrafes postérieures (6) vers l'extérieur de l'anneau (4) jusqu'à ce qu'elles entrent en contact avec la feuille d'agrafage (81) à travers la zone centrale (9), ce qui provoque leur déformation en acquérant la forme fermée qui comprime la prothèse vasculaire (94) et la paroi de l'aorte située sur la feuille d'agrafage (81) entre ses limites, fixant ainsi la prothèse vasculaire (94) à l'aorte au moyen de la feuille d'agrafage (81) et des agrafes postérieures (6).

2. Instrument pour l'agrafage chirurgical entre une prothèse vasculaire (94) et l'aorte, selon la revendication 1, **caractérisé en ce que** les moyens d'éjection des agrafes postérieures comprennent une tige d'actionnement (83) pour déplacer une série de coins coulissants (84) afin d'entraîner une série de plaques (85) qui éjectent les agrafes postérieures (6), lesdits coins coulissants (84) étant associés à des volets de retenue (86), lesquels décrivent un mouvement vers l'arrière pour libérer la feuille d'agrafage (81) de sa fixation par rapport au mécanisme d'agrafage pour le secteur aortique postérieur (80).

3. Instrument pour l'agrafage chirurgical entre une prothèse vasculaire (94) et l'aorte, selon l'une quelconque des revendications 1 à 2, **caractérisé en ce qu'**il comprend une pince auxiliaire (87) comprenant une traction (88) pour actionner le mécanisme d'agrafage pour le secteur aortique postérieur (80) incorporé dans l'anneau (4).

4. Instrument pour l'agrafage chirurgical entre une prothèse vasculaire (94) et l'aorte, selon la revendication 3, **caractérisé en ce que** la pince auxiliaire (87) comprend un adaptateur (89) à son extrémité distale qui entoure une canule de fixation (90) située dans l'anneau (4) lui-même, de sorte que l'adaptateur (89) contient un élément cylindrique d'entraînement (91) qui active le mécanisme d'agrafage pour le secteur aortique postérieur (80) incorporé dans l'anneau (4).

5. Instrument pour l'agrafage chirurgical entre une prothèse vasculaire (94) et l'aorte, selon l'une quelconque des revendications 1 à 4, **caractérisé en ce que** la feuille d'agrafage (81) comprend deux trous traversants (93) configurés pour incorporer des sutures supplémentaires.

6. Instrument pour l'agrafage chirurgical entre une prothèse vasculaire (94) et l'aorte, selon l'une quelconque des revendications 1 à 5, **caractérisé en ce que** le mécanisme de tir (50) comprend un engrenage mené (51) monté sur un arbre (52), qui déplace un coulisseau (53), pourvu d'un arbre à came télescopique (54) et d'un arbre à came limiteur (55), de sorte que le coulisseau (53), au moyen d'un arbre d'accouplement (56), permet la rotation d'une fourche (57) qui est associée à un cliquet d'entraînement (58), monté sur un arbre (59) et pourvu d'un ressort de rappel (60), lequel favorise la rotation du magasin rotatif (20), de sorte que le coulisseau (53) comporte une dernière section dans laquelle, une fois la rotation terminée et le positionnement correct du magasin rotatif (20) assuré au moyen d'un dispositif de fixation (61), l'arbre à came télescopique (54) vient en butée contre le coulisseau (53) lui-même et est entraîné avec celui-ci jusqu'à ce que ledit arbre à came télescopique (54) exerce la pression nécessaire, favorisant finalement l'activation de leviers de tir (62) qui déplacent des patins à crémaillère (63), de sorte que ces patins à crémaillère (63) déplacent des leviers dentés (95) qui déplacent des plaques coulissantes (96) qui éjectent les agrafes antérolatérales (5) pour leur déformation en forme de B.

7. Instrument pour l'agrafage chirurgical entre une prothèse vasculaire (94) et l'aorte, selon la revendication 6, **caractérisé en ce qu'**il comprend un mécanisme complémentaire comprenant un ressort (64) configuré pour déplacer un coin de friction (65), associé par un arbre (66) à une butée dynamique (67), laquelle est fixée au moyen d'un pivot oscillant (68) au dispositif de fixation (61) qui détermine la position du système mécanique pour permettre des mouvements de déverrouillage, de course de la butée dynamique et de verrouillage.

8. Instrument pour l'agrafage chirurgical entre une prothèse vasculaire (94) et l'aorte, selon l'une quelconque des revendications 1 à 7, **caractérisé en ce que** le mécanisme de compression-décompression (45) comprend des crémaillères guidées (46) réunies entre elles par des colonnes de liaison (47) configurées pour faire tourner des excentriques de traction (48), fournissant un mouvement d'avancement de la tête (3) afin d'exercer la compression des tissus à agrafer, et comprend en outre une came de butée (49) pour limiter le mouvement de décompression.

9. Instrument pour l'agrafage chirurgical entre une prothèse vasculaire (94) et l'aorte, selon l'une quelconque des revendications 1 à 8, **caractérisé en ce que** le mécanisme d'ancrage entre la tête et l'anneau (37) comprend des engrenages menés (38) réunis entre eux par un arbre solidaire (39) pour déplacer des crémaillères d'entraînement (40), lesquelles déplacent des supports de coins (41) à mouvement limité par des arbres limiteurs (42), favorisant finalement le déplacement des supports de coins (41), lesquels poussent des galets rétractables (43) en les introduisant dans les cavités latérales du port d'ancrage (44) et empêchant leur désengagement.

10. Instrument pour l'agrafage chirurgical entre une prothèse vasculaire (94) et l'aorte, selon l'une quelconque des revendications 1 à 9, **caractérisé en ce que** les trous périphériques (72) sont disposés en formant deux circonférences parallèles dans le corps cylindrique (69) de l'anneau (4).

11. Instrument pour l'agrafage chirurgical entre une prothèse vasculaire (94) et l'aorte selon l'une quelconque des revendications 1 à 10, **caractérisé en ce que** le corps cylindrique (69) comprend une rainure périphérique (71) configurée pour fixer la prothèse vasculaire (94) au moyen d'un élément de fixation amovible.

12. Instrument pour l'agrafage chirurgical entre une prothèse vasculaire (94) et l'aorte selon l'une quelconque des revendications 1 à 11, **caractérisé en ce que** le port d'ancrage (44) comprend une bague (73) pour entourer le corps cylindrique (69) de l'anneau (4) avec une capacité de translation rotative autour du corps cylindrique (69) et qui est fixée audit corps cylindrique (69) au moyen d'un écrou de serrage (74) ; il comprend en outre un coin d'indexation (75) qui, en combinaison avec un engrenage denté (76) du corps cylindrique (69), assure le positionnement correct de la tête (3) dans les différentes positions d'agrafage.

13. Instrument pour l'agrafage chirurgical entre une prothèse vasculaire (94) et l'aorte selon la revendication 12, **caractérisé en ce que** le coin d'indexation (75) comprend un ressort (77), logé dans son manchon correspondant (78), dans lequel une goupille de fixation (79) sert de butée pour le manchon (78), limitant la compression du ressort (77).

14. Instrument pour l'agrafage chirurgical entre une prothèse vasculaire (94) et l'aorte selon l'une quelconque des revendications 1 à 13, **caractérisé en ce que** les moyens internes de transmission de la tige comprennent une série de câbles de transmission (17) et leurs conduits de logement correspondants (18).

15. Instrument pour l'agrafage chirurgical entre une prothèse vasculaire (94) et l'aorte selon la revendication 14, **caractérisé en ce que** la tête (3) comprend un boîtier de mécanisme (92) qui incorpore une bobine d'actionnement du mécanisme d'ancrage (24), une bobine d'actionnement du mécanisme de compression-décompression (25) et une bobine d'actionnement du mécanisme de tir (26), de sorte que chacune des bobines (24, 25, 26) est associée aux câbles de transmission (17) de la tige (2), et active le mécanisme d'ancrage entre la tête et l'anneau (37), le mécanisme de compression-décompression (45) et le mécanisme de tir (50) des agrafes antérolatérales (5).

16. Instrument pour l'agrafage chirurgical entre une prothèse vasculaire (94) et l'aorte selon l'une quelconque des revendications 1 à 15, **caractérisé en ce que** le corps (19) de la tête (3) présente une courbure de la surface de contact alignée avec l'anneau (4) en respectant la morphologie de la circonférence de la section aortique.

17. Instrument pour l'agrafage chirurgical entre une prothèse vasculaire (94) et l'aorte selon l'une quelconque des revendications 1 à 16, **caractérisé en ce que** le corps (19) de la tête (3) comprend des rainures (22) configurées pour accompagner l'éjection des agrafes antérolatérales (5) à la distance optimale pour une déformation correcte en forme de B.
